# EUROPEAN PATENT SPECIFICATION

(11) **EP 3 666 322 B1**
(45) Date of publication and mention of the grant of the patent: **21.02.2024**
(21) Application number: 18842960.9
(22) Date of filing: 01.08.2018
(51) Int. Cl.: A61M 25/01, A61F 2/24, A61F 2/962

(54) **CARDIAC VALVE DELIVERY CATHETER AND DELIVERY SYSTEM**
HERZKLAPPENFREISETZUNGSKATHETER UND FREISETZUNGSSYSTEM
CATHÉTER DE POSE DE VALVULE CARDIAQUE ET SYSTÈME ASSOCIÉ

(30) Priority: 10.08.2017 CN 201710682415
(43) Date of publication of application: 17.06.2020
(73) Proprietor: Shanghai Microport Cardioflow Medtech Co., Ltd., Shanghai 201203 (CN)
(72) Inventor: LI, Longfei, Shanghai 201203 (CN); GUI, Baozhu, Shanghai 201203 (CN); LIU, Ming, Shanghai 201203 (CN); LIU, Shihong, Shanghai 201203 (CN); CHEN, Guoming, Shanghai 201203 (CN); LI, Yu, Shanghai 201203 (CN)
(74) Representative: Lavoix
(86) International application number: PCT/CN2018/097888
(87) International publication number: WO 2019/029404

(56) References cited:
- WO-A1-2017/027161
- WO-A1-2017/040269
- CN-A- 102 500 041
- CN-A- 103 491 905
- CN-A- 103 655 004
- CN-A- 107 496 055
- US-A1- 2014 148 889
- US-A1- 2014 343 670
- US-A1- 2015 231 367
- US-A1- 2015 297 346
- US-A1- 2017 182 291
- US-A1- 2017 189 184

## Description

### TECHNICAL FIELD

The present application relates to the technical field of interventional medical treatment and, more specifically, to a cardiac valve delivery catheter and a delivery system including the delivery catheter.

### BACKGROUND

In recent years, minimally invasive artificial cardiac valve replacement has attracted increasing attention. However, currently, there are still many problems in the technology related to cardiac valve delivery systems. For example, to improve delivery performance of an interventional valve delivery system for smoothly passing through an aorta, especially an aortic arch, and to improve coaxial performance of the delivery system with an annulus at the initial release of the delivery system, a conveyor is usually designed to be more flexible. In this way, the foregoing performances can be improved. However, when the valve is deployed, it is prone to movement, lowering stability in release of the valve. In addition, when the valve is retrieved before the valve is deployed completely by the delivery system, it is likely that an outer tube of the delivery system cannot bear forces, and finally, the outer tube is compressed. Consequently, the delivery system cannot successfully retrieve the valve.

To achieve a retrieval function before the valve is completely deployed, a delivery system designed to be relatively rigid is used in the related art, and even inner and outer tubes and a delivery system segment sheath for holding and compressing the valve are designed with reinforcing ribs. In this way, the stability in deploy of the valve is improved and a function of retrieving and then releasing again the valve in human body is achieved. However, it is not easy for the conveyor to pass through large curved pathways, especially aortic arches with spatial three-dimensional bending characteristics. If the conveyor forcibly passes through the pathways, a severe consequence of scratching or piercing a blood vessel is easily caused. In addition, difficulty in locating the delivery system segment sheath, for holding and compressing the valve and for retrieving the valve, makes it difficult to ensure a coaxiality with an annulus root, resulting in reduced quality in deploy of the valve.

US 2014/148889 A1 describes a distal tip for a delivery catheter.

WO 2017/027161 A1 describes a system and method for deflecting a delivery catheter.

US 2014/343670 A1 describes a prosthetic heart valve delivery apparatus.

US 2017/189184 A1 describes systems for implanting an endoprosthesis.

US 2015/297346 A1 describes a hinged transcatheter prosthetic heart valve delivery system.

### SUMMARY OF THE INVENTION

In view of this, it is desired to provide a cardiac valve delivery catheter and delivery system to solve a problem that it is difficult for an exisiting conveyor to retrieve an incompletely deployed valve in human body without affecting quality in deploy of the valve.

The invention relates to a cardiac valve delivery catheter according to claim 1.

Additional features of the cardiac valve delivery catheter are defined in claims 2 to 6.

Accordingly, in a further aspect, the present application provides a cardiac valve delivery system according to claim 7.

According to the foregoing cardiac valve delivery catheter and delivery system, the stent segment sheath includes the metal reinforced sheath, so that the stent segment sheath can more easily pass through multi-direction curved pathways and has a good compressive property. Therefore, when the valve is deployed partially, the stent segment sheath still has good radial tension. In addition, an elastic deformable metal tube with a hollow structure is disposed inside the distal end of the delivery segment outer tube, and the delivery segment outer tube can retain a particular curved shape after entering the ascending aorta, so that coaxiality between the stent segment sheath and an annulus in the ascending aorta can be adjusted, to ensure the effect in deploy of the valve, therefore retrieval and deploy of the valve in the human body can be achieved without affecting the effect in deploy of the valve.

### BRIEF DESCRIPTION OF THE DRAWINGS

To describe the technical solutions in the embodiments of the present application or in the related art more clearly, the following briefly describes the accompanying drawings required in the embodiments or the related art. Apparently, the accompanying drawings in the following description show merely some embodiments of the present application, and a person of ordinary skill in the art may derive drawings in other embodiments from these accompanying drawings without creative efforts.
FIG. 1 is a schematic structural diagram of a delivery system according to an implementation;
FIG. 2 is a schematic assembly diagram of a delivery catheter, a stability tube and a guide sheath according to another implementation;
FIG. 3 is a schematic structural diagram of an inner tube assembly according to another implementation;
FIG. 4 is a diagram of a status in which a valve is deployed from a fixing head of an inner tube assembly according to another implementation;
FIG. 5 is a schematic diagram showing a connection relationship between the handle structure and an inner tube assembly and between the handle structure and an outer tube assembly according to another implementation;
FIG. 6 is a schematic diagram showing sleeve connections between an outer tube assembly, a stabilization tube assembly, and a guide sheath, where a stabilization tube round head is disposed at a distal end of a stabilization tube;
FIG. 7 is a schematic structural diagram of a stent segment sheath according to another implementation;
FIG. 8 is a schematic structural diagram of an inner tube assembly according to another implementation;
FIG. 9 is a schematic extension diagram of a connection portion and a hollow portion of a hollow tubular structure according to another embodiment;
FIG. 10 is a schematic structural diagram of a hollow tubular structure according to another embodiment;
FIG. 11 is a schematic structural diagram of a hollow structure with spiral reinforcing ribs according to another embodiment;
FIG. 12 is a schematic structural diagram of a hollow structure with straight reinforcing ribs according to another embodiment;
FIG. 13 is a schematic structural diagram of a hollow tubular structure according to another embodiment;
FIG. 14 is a schematic three-dimensional structural diagram of the hollow tubular structure in FIG. 9; and
FIG. 15 is a schematic diagram showing projections on a cross section of the hollow tubular structure in FIG. 14.

### DETAILED DESCRIPTION

For ease of understanding of the present application, the present application is described more fully below with reference to the related accompanying drawings. Preferred embodiments of the present application are provided in the accompanying drawings. However, the present application may be implemented in many different forms and is not limited to the embodiments described herein. On the contrary, these embodiments are provided so that disclosed content of the present application are more thorough and complete.

It should be noted that "distal end" and "proximal end" are used as position words. The position words are commonly used in the field of interventional medical devices. The "distal end" indicates an end away from an operator during an operation, and the "proximal end" indicates an end near the operator during the operation. An axial direction refers to a direction parallel to a line connecting centers of far and proximal ends of a medical device, and a radial direction refers to a direction perpendicular to the foregoing axial direction.

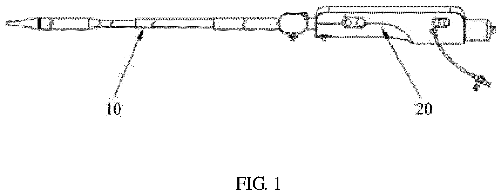

Referring to FIG. 1, a cardiac valve delivery system according to an embodiment of the present application includes a delivery catheter 10 and a handle 20. A proximal end of the delivery catheter 10 is connected to the handle 20.

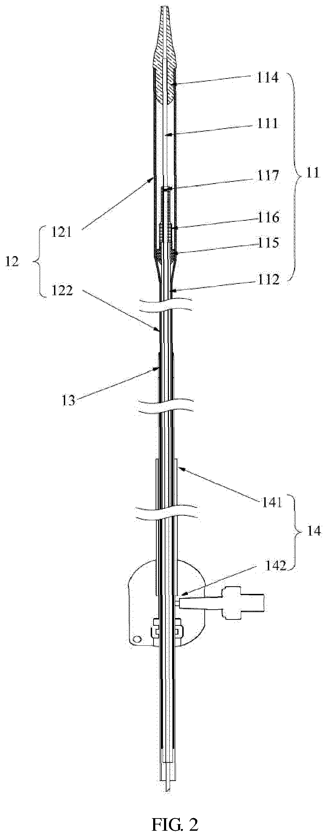

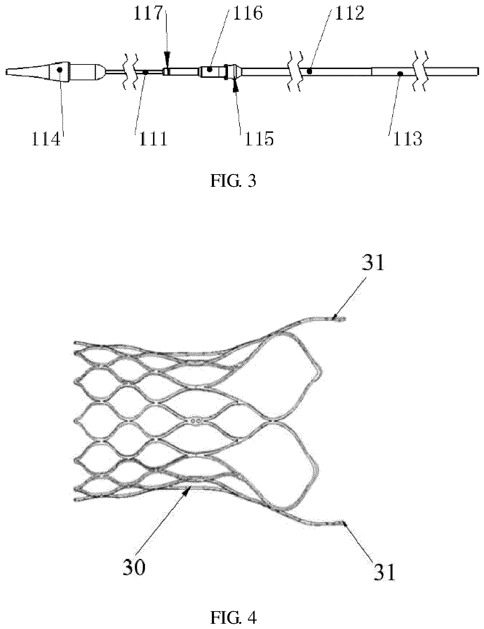

Referring to both FIG. 2 and FIG. 3, in some embodiments, the delivery catheter 10 includes an inner tube assembly 11 and an outer tube assembly 12. A relative movement between the inner tube assembly 11 and the outer tube assembly 12 can be achieved by the operation on handle 20. Specifically, a cavity is axially defined in the outer tube assembly 12, and the inner tube assembly 11 can move relative to the outer tube assembly 12 in the cavity along an axial direction to cause a displacement. The inner tube assembly 11 includes an inner core tube 111, an inner tube 112, a conical head 114, and a fixing head 115. A distal end of the inner core tube 111 is connected to a proximal end of the conical head 114, a proximal end 15 of the inner core tube 111 is connected to the handle, the inner tube 112 sleeves over the inner core tube 111, and a distal end of the inner tube 112 is connected to the fixing head 115, and a proximal end of the inner tube 112 is connected to the handle. A valve 30 is detachably connected to the fixing head 115 through a structure such as a stent. During delivery, the valve 30 is accommodated in an annular cavity formed between the inner core tube 111 and the outer tube assembly 12. After a predetermined deploy position is reached, a relative displacement between the inner tube assembly 11 and the outer tube assembly 12 occurs due to the operation on handle 20, and the valve 30 is pushed completely out of the distal end of the outer tube assembly 12, so that the valve 30 is detached and deployed from the fixing head 115.

In the foregoing embodiment, the inner core tube 111 and the inner tube 112 are made of a polymer material and respectively formed by weaving. The materials used at the distal end and the proximal end of the inner tube 112 may be different. For example, a relatively flexible material may be used at the distal end of the inner tube 112, so that the distal end can more easily pass through an aortic arch, while a relatively hard material may be used at the proximal end of the inner tube 112 to improve an overall tensile and/or compressive property of the inner tube 112. Certainly, to improve overall delivery performance of the inner tube assembly 11, the proximal end of the inner tube 112 may be further connected to a metal reinforcing tube 113. It may be understood that, the metal reinforcing tube 113 also sleeves over the inner core tube 111, and a proximal end of the metal reinforcing tube 113 is connected to the handle.

In some embodiments, the outer tube assembly 12 includes a stent segment sheath 121 capable of accommodating the valve 30 and includes a delivery segment outer tube 122 connected to a proximal end of the stent segment sheath 121. It may be understood that, to increase an accommodation space between the inner core tube 111 and the stent segment sheath 121 to better accommodate the valve 30, a diameter of the inner core tube 111 is slightly less than a diameter of the inner tube 112, and a diameter of the stent segment sheath 121 is greater than a diameter of the delivery segment outer tube 122, so that a cavity for accommodating the valve 30 can be formed.

In some embodiments, the conical head 114 is designed to be streamlined for achieving better traceability. In addition, the proximal end of the conical head 114 extends into the stent segment sheath 121 from an opening at a distal end of the stent segment sheath 121, to minimize a flaring formed by an end surface of the distal end of the stent segment sheath 121 and the conical head 114 when the stent segment sheath 121 passes through the aortic arch.

Referring to FIG. 4, in some embodiments, a clamping groove is defined on the fixing head 115, so that a lug 31 of the valve 30 is clamped in the clamping groove to achieve a good axial limiting. When the valve 30 is accommodated in the stent segment sheath 121, the stent of the valve 30 can slide relative to the stent segment sheath 121 by sliding the inner tube 112. When sliding out of the stent segment sheath 121, the valve 30 expands and results in that the lug 31 of the valve 30 is no longer clamped in the clamping groove, so that the valve 30 is detached and deployed from the fixing head 115. Certainly, to better support the valve and prevent the valve 30 from twisting when the valve 30 is mounted and retrieved, a step structure 116 is disposed at one end of the fixing head 115 connected to the inner core tube 111 for transition. An outer diameter of the step structure 116 is designed to a size matching an inner diameter of the valve that is compressed and held, so that the valve can be supported and prevented from twisting.

In some embodiments, when the proximal end of the conical head 114 is received in the distal end of the stent segment sheath 121, the valve 30 is completely accommodated in the stent segment sheath 121, and a proximal end of the fixing head 115 abuts against the proximal end of the stent segment sheath 121 and is accommodated in the stent segment sheath 121. In some other embodiments, there may be a gap between the proximal end of the fixing head 115 and the proximal end of the stent segment sheath 121. In this way, it is ensured that the valve 30 is completely accommodated in the cavity between the inner core tube 111 and the stent segment sheath 121, and a case in which the service life is affected because the fixing head 115 abuts against a tube wall of the proximal end of the stent segment sheath 121 for a long time is prevented. On this basis, the proximal end of the fixing head 115 and the proximal end of the stent segment sheath 121 may be designed as arc-shaped transition sections having matching shapes.

Referring to both FIG. 2 and FIG. 3, a developing ring 117 is disposed on the inner core tube 111. During the delivery, the exposed developing ring 117 out of the opening at the distal end of the stent segment sheath 121 can be observed through a developing device, to observe a relative position between the inner core tube 111 and the stent segment sheath 121. Because a proximal end of the valve 30 is fixed on the fixing head 115, a relative installation position of the proximal end of the valve 30 with the developing ring 117 is predetermined, so that when the developing ring 117 is exactly located at the opening at the distal end of the stent segment sheath 121 and observed, a degree at which the valve 30 is deployed from the distal end of the stent sheath can be determined. For example, a distance between the proximal end of the valve 30 and the developing ring 117 is half of an axial length of the valve 30, and when the developing ring 117 moves to the opening at the distal end of the stent segment sheath 121 and is observed, it may be determined that the degree at which the valve 30 is deployed from the distal end of the stent segment sheath 121 is exactly half of the degree of a complete deploy. To conveniently identify the incompletely deployed valve 30 during retrieval in human body, the distance between the developing ring 117 and the proximal end of the valve 30 is 1/4 of the axial length of the valve 30. Therefore, when the developing ring 117 is located at the opening at the distal end of the stent segment sheath 121, the valve 30 is deployed from the stent segment sheath 121 by 3/4. In this way, most of the valve 30 can be deployed to observe a deploy effect. When a deploy position is undesired, 1/4 of the valve 30 remained in the stent segment sheath 121 can ensure that the valve 30 is stably retrieved in the stent segment sheath 121 when being pulled by the inner core tube 111, and the following situation can be avoided: a large deviation in coaxiality between the valve 30 and the stent segment sheath 121 is caused during retrieval of the valve 30 because a very small part of the valve 30 is retained in the stent segment sheath 121, affecting an effect of re- deploy.

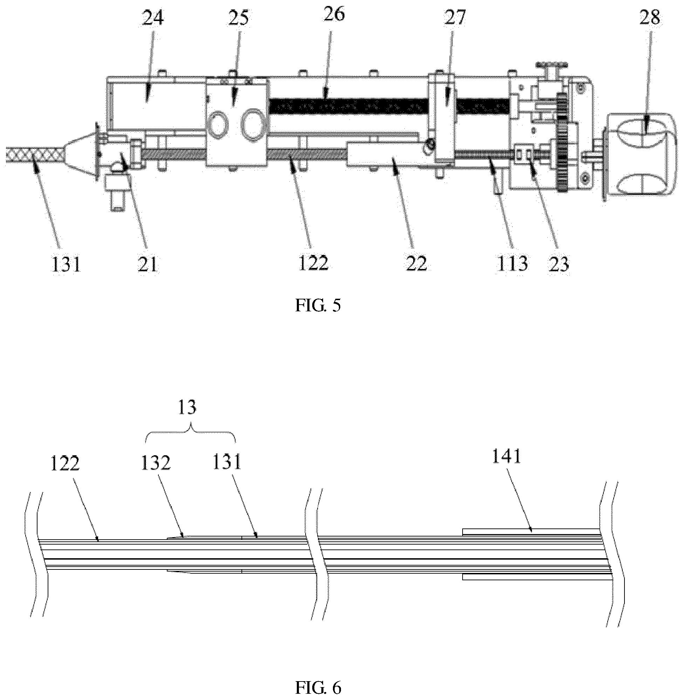

It may be understood that, in some embodiments, a driving mechanism is disposed in the handle 20, to drive the inner tube assembly 11 to axially move relative to the outer tube assembly 12. The driving mechanism may provide a manual or electric drive, or a manual and electric hybrid drive. A hybrid drive is currently used as an example to further describe the driving mechanism. Referring to FIG. 5, an electric part of the driving mechanism includes a motor 24 and a screw rod 26 linked with an output shaft of the motor 24. The screw rod 26 screw-drives a moving member 27, so that the moving member 27 drives a delivery segment outer tube 122 fixedly connected to the moving member 27 to axially move relative to the metal reinforcing tube 113 of the inner tube assembly 11. A control module 25 for controlling the motor 24 is disposed on the handle 20. A manual knob 28 is linked with the screw rod 26 through gear engagement or in another manner, so that the moving member 27 can also move along the screw rod 26 by means of the manual knob 28. Certainly, it may be understood that, the moving member 27 may be connected to a proximal end of the delivery segment outer tube 122 through an outer tube fixing seat 22. Correspondingly the metal reinforcing tube 113 is fixed in the handle 20 through an inner tube fixing seat 23. The fixing seat 23 may be fixed in a recess on a housing of the handle 20 through clamping or in another manner, so that the entire inner tube assembly 11 is fixed relative to the handle 20.

Still referring to FIG. 5, in some embodiments, to effectively support the inner tube assembly 11 after the inner tube assembly 11 is inserted into the delivery segment outer tube 122 of the outer tube assembly 12, a stabilization tube assembly 13 for the outer tube assembly 12 to pass through is installed at a front end of the handle 20 through a stabilization tube fixing seat 21. It may be understood that, the stabilization tube fixing seat 21 may be fixed in the handle 20 through clamping or welding or in another manner, to support the entire delivery system, thereby facilitating delivery of the delivery system and improving deploy stability of the valve 30. Further, referring to FIG. 6, the stabilization tube assembly 13 includes a stabilization tube 131 and a stabilization tube round head 132 located at a distal end of the stabilization tube 131. A connection between the stabilization tube round head 132 and the stabilization tube 131 may be a fixed connection through hot melting or the like. The stabilization tube round head 132 achieves a smooth transition between the stabilization tube 131 and the delivery segment outer tube 122 of the outer tube assembly 12 to avoid scratching a blood vessel wall.

In some embodiments, referring to FIG. 2, after the outer tube assembly 12 is inserted into the stabilization tube 131, when the delivery system delivers the valve into the human body, the stabilization tube 131 is arranged to pass through a guide sheath 141 of an inline delivery catheter sheath 14 in advance, and the inline delivery catheter sheath 14 is arranged to move toward the distal end along the stabilization tube 131 until a distal end of the guide sheath 141 abuts against the root of the proximal end of the stent segment sheath 121. Under the control of a sheath core 142, the stabilization tube 131 as well as the inner tube assembly 11 and the outer tube assembly 12 which are inside the stabilization tube 131 is bent with the guide sheath 141, and the valve delivery system enters the human body from a puncture port.

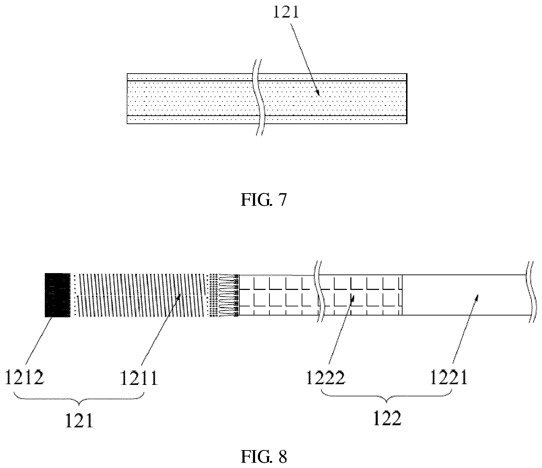

Referring to FIG. 7, in some embodiments, the stent segment sheath 121 is made of a high-strength polymer material, so that the stent segment sheath 121 has relatively high pressure resistance, thereby ensuring that when the valve 30 is retrieved into the stent segment sheath 121in the human body from an incompletely deployed status, the stent segment sheath 121 is not deformed by extrusion.

Referring to FIG. 8, in some embodiments, the stent segment sheath 121 is formed by coating a polymer coating on a surface of a metal reinforced sheath 1211, to improve a tensile and/or compressive property of the stent segment sheath 121. To reduce resistance in mounting and retrieving the valve 30 and thus improve the stability in deploy of the valve 30, an elastic extension section 1212 is disposed at a distal end of the metal reinforced sheath 1211. When the valve 30 is deployed, the elastic extension section 1212 may perform adaptable elastic expansion, which also facilitates retrieval of the valve 30.

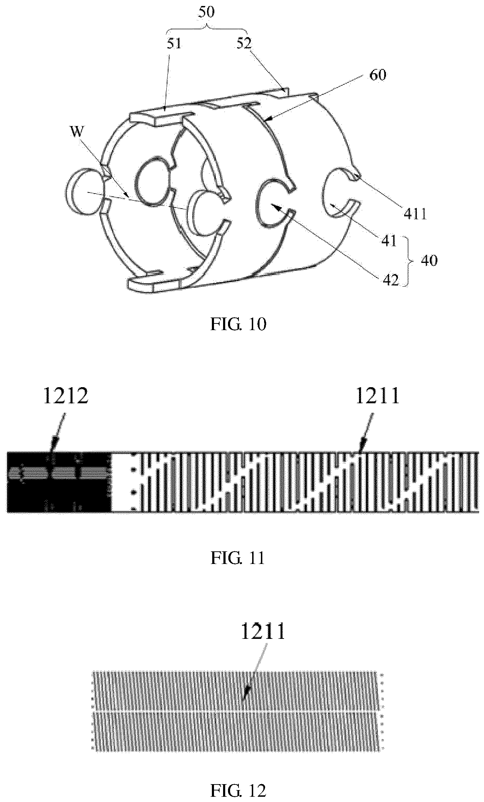

Further, the metal reinforced sheath 1211 has a hollow structure with reinforcing ribs, to achieve that the metal reinforced sheath 1211 has a relatively high tensile and/or compressive property and can be bended flexibly as well. It may be understood that, a specific configuration of the reinforcing ribs of the hollow structure may be correspondingly adjusted based on flexibility required by the metal reinforced sheath 1211. As shown in FIG. 11, a main body of the metal reinforced sheath 1211 has a hollow structure with spiral reinforcing ribs. In other words, a spiral part not hollowed is formed on the metal reinforced sheath 1211, the part forms the spiral reinforcing ribs, and the remaining part is formed with hollow slots distributed evenly. In this way, a relatively high tensile and/or compressive capability can be achieved, and a spatial multi-directional bending capability can be achieved, and thus a better adaption to the aortic arch can be achieved, which prevents from scratching and puncturing a blood vessel when the aortic arch is passed through. Certainly, in other embodiments, as shown in FIG. 12, the main body of the metal reinforced sheath 1211 may alternatively have a hollow structure with straight reinforcing ribs. In other words, a straight part not hollowed is formed on the metal reinforced sheath 1211, the part forms the straight reinforcing ribs, and the remaining part is formed with hollow slots distributed evenly.

Still referring to FIG. 8, in some embodiments, the delivery segment outer tube 122 includes a polymer tube 1221 and a hollow tubular structure 1222 (for ease of understanding, herein, only a part of the polymer tube 1221 is shown and the hollow tubular structure 1222 is exposed). The hollow tubular structure 1222 is disposed at one end of the polymer tube 1221 near the stent segment sheath 121. The delivery segment outer tube 122 further includes a metal braided tubular structure (not shown). The metal braided tubular structure and the hollow tubular structure 1222 are both disposed in a tube wall of the polymer tube.

In some embodiments, the metal braided tubular structure is disposed in the entire delivery segment outer tube 122, and the hollow tubular structure 1222 is disposed only in a segment of the delivery segment outer tube 122 near the stent segment sheath 121.

The polymer tube 1221 includes a first segment and a second segment. The hollow tubular structure 1222 is disposed in the first segment. Hardness of a material of the second segment is higher than that of the first segment. In this way, a segment including the hollow tubular structure has a better tensile and bending property, and overall connection strength of the delivery segment outer tube 122 is improved.

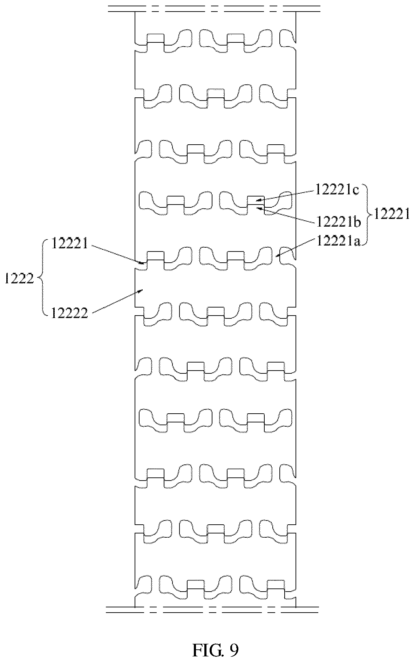

Referring to FIG. 9, in some embodiments, the hollow tubular structure 1222 is a hollow structure cut from a metal tube. The hollow structure includes a plurality of axially spaced hollow portions 12221. A connection portion 12222 is formed between adjacent hollow portions 12221. The hollow portions 12221 are disposed circumferentially on the metal tube. The hollow portions 12221 and the connection portions 12222 are disposed in parallel. There is a plurality of connecting ribs and a plurality of hollow slots circumferentially arranged on of each hollow portion 12221, the connecting ribs are spaced by the hollow slots, the connecting rib is configured to connect two axially adjacent connection portions 12222, and an edge of the hollow slot has an arc-shaped curve profile. Because the connecting ribs are spaced by the hollow slots, two connection portions can be bent towards each other with a connection to the connecting ribs. It may be understood that, there are an even number of hollow slots; in other words, there are also an even number of connecting ribs spaced by the hollow slots.

Specifically, in some embodiments, the hollow portion 12221 includes a plurality of connecting ribs 12221a for connecting adjacent connection portions 12222, and the hollow portion 12221 further includes a plurality of W-shaped hollow slots. The W-shaped hollow slots and the connecting ribs 12221a are spaced circumferentially on the hollow tubular structure 1222. Preferably, axially adjacent W-shaped hollow slots and connecting ribs 12221a are axially staggered. To be specific, projections of axially adjacent W-shaped hollow slots on a same vertical projection plane do not completely overlap, and projections of axially adjacent connecting ribs 12221a on a same vertical projection plane do not completely overlap. Further, distances between the projections of the axially adjacent connecting ribs 12221a on the same vertical projection plane are equal. Lines connecting a plurality of axially adjacent connecting ribs 12221a are spiral, so that the hollow tubular structure 1222 has a hollow structure with spiral reinforcing ribs. Herein, the "axially adjacent connecting ribs 12221a" are two connecting ribs 12221a closest to two ends of the connection portion 12222 along an axial direction of the hollow tubular structure 1222.

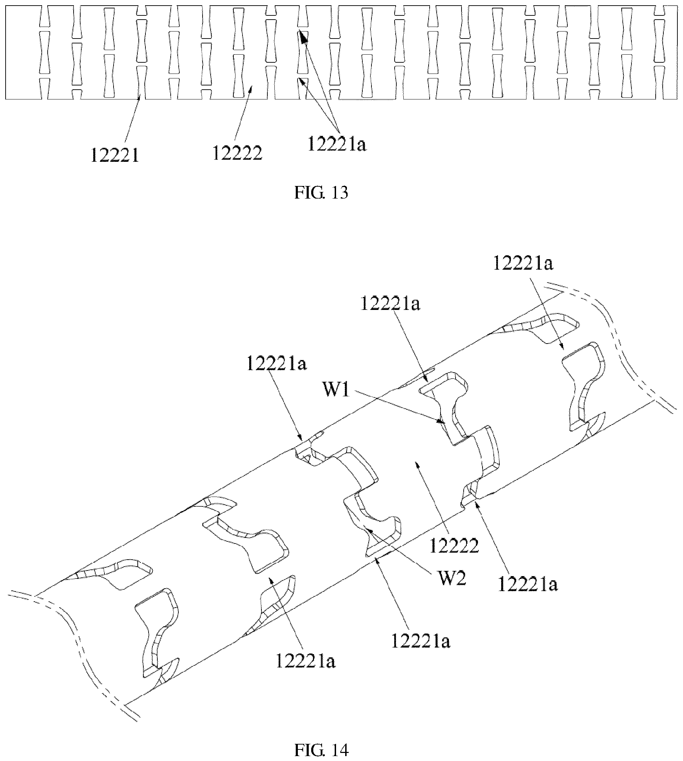

It may be understood that, with reference to FIG. 13, the hollow slots in the hollow portion 12221 may also be linear. The hollow portion has two opposite connecting ribs 12221a in the circumferential direction. In this way, adjacent connection portions 12222 may rotate by a particular angle about an axis defined by a line connecting the two connecting ribs 12221a, so that the hollow tubular structure 1222 can be axially bent. This hollow structure not only satisfies a requirement on tensile and/or compressive property of the delivery segment outer tube 122, but also enables the delivery segment outer tube 122 to have a very good bending property, thereby reducing movement during deploy of the valve 30 and improving the stability in deploy of the valve 30. In addition, the delivery segment outer tube 122 having the hollow structure would be subject to a particular bending deformation after entering the ascending aorta, and since the proximal end of the delivery segment outer tube 122 is in contact with and supported by a blood vessel wall, the stent segment sheath 121 and an annular root plane of the valve 30 can maintain coaxiality by adjusting the handle 20, to improve deploy effect of the valve 30.

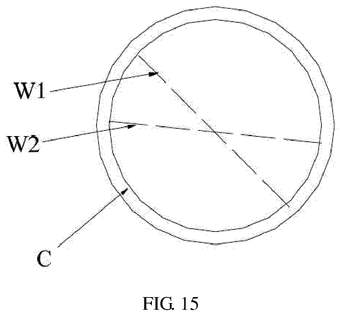

In some embodiments, lines connecting two connecting ribs 12221a on hollow portions 12221 at two ends of each connection portion 12222 are parallel to each other. In this structure, the connection portions 12222 forming the hollow tubular structure 1222 rotate in a same plane, and are bent in a two-dimensional plane, so that an adjustment requirement for maintaining the coaxiality between the stent segment sheath 121 and the annular root plane of the valve 30 can be satisfied. It may be understood that, in the circumferential direction of the connection portion 12222, there is an angle between the lines connecting the two connecting ribs 12221a of the hollow portions 12221 at the two ends of each connection portion 12222. Specifically, referring to both FIG. 14 and FIG. 15, an example in which each hollow portion 12221 has two hollow slots is provided. Projections of a line W1 connecting two opposite connecting ribs 12221a at one end of the connection portion 12222 and of a line W2 connecting two opposite connecting ribs 12221a at the other end of the connection portion 12222 on a cross section C of the connection portion 12222 intersect each other. To be specific, adjacent connection portions 12222 are respectively bent about different rotation axes W1 and W2. Because the connection portions 12222 are bent in different directions, the hollow tubular structure 1222 including the plurality of connection portions 12222 can be bent toward a plurality of directions. Therefore, by virtue of this structure, the hollow tubular structure 1222 can be bent in any direction in a three-dimensional space, so that the stent segment sheath 121 connected to the distal end of the delivery segment outer tube 122 can very easily pass through the aortic arch, and the adjustment requirement for maintaining the coaxiality between the stent segment sheath 121 and the annular root plane of the valve 30 is satisfied, thereby improving the effect in deploy of the valve 30.

In the foregoing embodiment, when two adjacent connection portions 12222 respectively rotate about two connection lines perpendicular to each other, to be specific, projections of the connection lines W1 and W2 on the cross section C of the connection portion 12222 are perpendicular to each other, the connection portions 12222 have relatively large rotation angles toward various dimensions, so that the hollow tubular structure 1222 can achieve a larger bending effect. Therefore, the distal end of the delivery segment outer tube 122 can achieve different bending properties by adjusting spiral rotation angles of two connecting ribs 12221a about the axial direction of the hollow tubular structure 1222, so that the adjustment requirement for maintaining the coaxiality with the annular root plane of the valve 30 can be satisfied.

Further, as shown in FIG. 9, the hollow portion 12221 further includes a clamping piece 12221b and a retaining groove 12221c disposed circumferentially. In this embodiment, the retaining groove 12221c is a part of the W-shaped hollow slot. The clamping piece 12221b and the retaining groove 12221c are located on a midperpendicular of a line connecting two connecting ribs 12221a. The clamping piece 12221b is limited in the retaining groove 12221c in the circumferential direction of the hollow portion 12221. When adjacent connection portions 12222 perform relative rotation about a rotation axis defined by a line connecting two connecting ribs 12221a, the clamping piece 12221b can move in the limiting groove 52 and has a limiting position. When the adjacent connection portions 12222 rotate about a line connecting the two connecting ribs 12221a and when the limiting position is reached, the top of the clamping piece 12221b abuts against the bottom of the retaining groove 12221c, to effectively prevent further rotation and bending of the connection portions 12222. In this way, the hollow tubular structure 1222 is allowed to be bent by a particular degree, and good support is ensured. In addition, the clamping piece 12221b is limited in the retaining groove 12221c in the circumferential direction, which effectively prevents the connection portions 12222 from twisting in the circumferential direction when the hollow tubular structure 1222 is bent, thereby preventing the connection portions 12222 from twisting in the circumferential direction, which causes damage to the connecting ribs 12221a and avoid impact on an effect of rotation of the connection portions 12222 about a line connecting the connecting ribs 12221a.

Referring to FIG. 10, in some other embodiments, the hollow tubular structure 1222 is formed by a plurality of axially interlocked metal rings. Adjacent metal rings form interlocked movable connection portions 40 at connecting end surfaces; and the adjacent metal rings are rotatable about the movable connection portions 40 to bend the hollow tubular structure 1222.

In some embodiments, the movable connection portion 40 is implemented as a circular notch 41 and a circular protrusion 42 opposite disposed on two end surfaces of the metal rings. The circular notch 41 and the circular protrusion 42 are respectively located on radially opposite circumferences of the metal rings, and a size of the circular protrusion 42 is slightly less than that of the circular notch 41. A bayonet 411 for clamping the root of the circular protrusion 42 is formed at an opening of the circular notch 41, and a gap 60 for adjacent metal rings to rotate around the bayonet 411 is reversed at two sides of the bayonet 411 on the end surfaces of the metal rings. The gap 60 can meet a requirement for a rotation space between the metal rings. In addition, when a gap 60 at one side is closed by the rotation of the metal rings, the metal rings are restricted from rotating due to the abutting end surfaces of the metal rings, so that a bending degree of the hollow tubular structure 1222 can be controlled. It may be understood that, the metal rings perform axial rotation around the bayonet 411. Therefore, the gap 60 may have a particular oblique angle, to be specific, increases from a near side to a far side of the bayonet 411, so that the metal rings rotate to the limiting position, and end surfaces of two metal rings defining the gap 60 can be closely attached together, as a result, the metal rings can be supported better in a bending direction, thereby improving stability of the delivery segment outer tube 122.

In some embodiments, a circumferential limiting portion 50 is further disposed on the end surface of the metal ring; the circumferential limiting portion 50 and the movable connection portion 40 are spaced in the circumferential direction of the metal ring; and a relative deflection angle between the circumferential limiting portion 50 and the movable connection portion 40 is 90°. The circumferential limiting portion 50 limits the metal rings in the circumferential direction, to improve an anti-torsion property of the metal rings, thereby preventing torsion caused by circumferential rotation from damaging stability of the movable connection portion 40. In addition, when the relative deflection angle between the circumferential limiting portion 50 and the movable connection portion 40 in the circumferential direction of the metal ring is 90°, rotation of the metal rings around the movable connection portion 40 is not affected. To be specific, when the metal rings perform relative rotation around the rotation axis W, the circumferential limiting portion 50 limits the metal ring only in the circumferential direction, and the circumferential limiting portion 50 can shrink accordingly with the rotation of the metal rings due to since the circumferential limiting portion 50 is perpendicular to the movable connection portion 40.

In some embodiments, a first rotation axis and a second rotation axis respectively formed from rotations of two adjacent metal rings around the bayonet 411 are parallel. In other words, all the metal rings are bent in a same plane. In this structure, the hollow tubular structure 1222 can be bent and deformed in the two-dimensional plane, and the adjustment requirement for maintaining the coaxiality with the annular root plane of the valve 30 can be satisfied, thereby improving the stability in deploy of the valve 30.

In some implementations, a first rotation axis and a second rotation axis respectively formed from rotations of two adjacent metal rings around the bayonet 411 have their projections on the end surfaces of the metal rings which intersect each other. To be specific, two adjacent metal rings can rotate in different planes, so that the hollow tubular structure 1222 formed by the metal rings can be bent in at least a plurality of planes. In other words, in this structure, the hollow tubular structure 1222 can be bent in any direction in the three-dimensional space, so that the stent segment sheath 121 connected to the distal end of the delivery segment outer tube 122 can very easily pass through the aortic arch, and the adjustment requirement for maintaining the coaxiality between the stent segment sheath 121 and the annular root plane of the valve 30 is satisfied, thereby improving the effect in deploy of the valve 30. When rotation axes of the adjacent metal rings are perpendicular to each other, rotation angles of the metal rings in various dimensions are allowed to be relatively large in the three-dimensional space, therefore a great bend of the hollow tubular structure 1222 is achieved, thereby satisfying the adjustment requirement for maintaining the coaxiality with the annular root plane of the valve 30.

In some embodiments, the circumferential limiting portion 50 includes a limiting piece 51 and a limiting groove 52 whose relative deflection angles with the bayonet 411 are 90° in the circumferential direction of the metal ring. The limiting piece 51 is limited in the limiting groove 52 in the circumferential direction of the metal ring, and when adjacent metal rings perform relative rotation, the limiting piece 51 is movable in the limiting groove 52 and has a limiting position. At the limiting position, the top of the limiting piece 51 contacts the bottom of the limiting groove 52, to limit further relative movement of the metal rings. The limiting piece 51 matches the limiting groove 52, to implement good circumferential limiting to improve a circumferential anti-torsion property, thereby avoid damage to the movable connection portion 40. In addition, in this structure, the matching between the limiting piece 51 and the limiting groove 52 does not affect rotation of the metal rings.

The technical features of the foregoing embodiments may be combined randomly. For the purpose of brief description, all the possible combinations of the technical features in the foregoing embodiments are not described. However, as long as there is no contradiction in these combinations of the technical features, they should be considered as falling within the scope recorded in this specification.

## Claims

1. A cardiac valve delivery catheter (10), comprising an outer tube assembly (12) and an inner tube assembly (11), wherein the outer tube assembly (12) comprises a stent segment sheath (121) configured to accommodate a valve and a delivery segment outer tube (122) connected to a proximal end of the stent segment sheath (121); the inner tube assembly (11) is disposed in a cavity of the outer tube assembly (12), and the inner tube assembly (11) is axially movable relative to the outer tube assembly (12); and the delivery segment outer tube (122) comprises a polymer tube (1221) and a hollow tubular structure (1222) disposed in the polymer tube (1221);
wherein the hollow tubular structure (1222) is cut from a metal tube, and the hollow tubular structure (1222) comprises a plurality of hollow portions (12221) arranged in an axially spaced manner and connection portions (12222) each formed between two adjacent ones of the plurality of hollow portions (12221); an even number of connecting ribs (12221a) and an even number of hollow slots are arranged on each hollow portion (12221) in a circumferential direction of the hollow portion (12221); each of the connecting ribs (12221a) is disposed between two of the plurality of hollow slots; and the connecting ribs (12221a) are configured to connect two axially adjacent ones of the connection portions (12222);
wherein each of the hollow slots is W-shaped; each of the hollow portions (12221) further comprises a clamping piece (12221b) and a retaining groove (12221c) which are disposed along the circumferential direction; the retaining groove (12221c) is a part of a corresponding one of the hollow slots; the clamping piece (12221b) and the retaining groove (12221c) are located on a midperpendicular of a line connecting two adjacent ones of the connecting ribs (12221a); the clamping piece (12221b) is limited in the retaining groove (12221c) in the circumferential direction of the hollow portion (12221); and when adjacent ones of the connection portions (12222) perform relative rotation, the clamping piece (12221b) moves relative to the limiting groove (52) and has a limiting position;
wherein lines (W1, W2) each connecting two opposite connecting ribs (12221a) on a respective one of the hollow portions (12221) at two ends of each one of the connection portions (12222) have projections intersecting each other on a cross section (C) of the connection portion (12222);
wherein distances between projections of axially adjacent connecting ribs (12221a) on a same vertical projection plane are equal, and lines connecting the plurality of connecting ribs (12221a) that are distributed axially are spiral.

2. The cardiac valve delivery catheter (10) according to claim 1, wherein the stent segment sheath (121) comprises a metal reinforced sheath (1211), an elastic extension section (1212) connected to a distal end of the metal reinforced sheath (1211), and a polymer coating coated over an outer side of the metal reinforced sheath (1211) and the elastic extension section (1212); wherein the metal reinforced sheath (1211) has a hollow structure with spiral reinforcing ribs.

3. The cardiac valve delivery catheter (10) according to claim 1, wherein the even number is two.

4. The cardiac valve delivery catheter (10) according to claim 1, wherein edges of each of the hollow slots are all arc-shaped curves.

5. The cardiac valve delivery catheter (10) according to claim 1, wherein the delivery segment outer tube (122) further comprises a metal braided tubular structure, and both the metal braided tubular structure and the hollow tubular structure (1222) are disposed in a tube wall of the polymer tube (1221);
wherein the metal braided tubular structure is disposed in all sections of the delivery segment outer tube (122), and the hollow tubular structure (1222) is disposed in some of the sections of the delivery segment outer tube (122) near the stent segment sheath (121).

6. The cardiac valve delivery catheter (10) according to claim 1, wherein the delivery segment outer tube (122) further comprises a metal braided tubular structure, and both the metal braided tubular structure and the hollow tubular structure (1222) are disposed in a tube wall of the polymer tube (1221);
wherein the polymer tube (1221) comprises a first segment and a second segment which are connected to each other, the hollow tubular structure (1222) is disposed in the first segment, and the second segment has a hardness greater than a hardness of the first segment.

7. A cardiac valve delivery system, comprising a handle (20) and the delivery catheter (10) according to any one of claims 1 to 6, the delivery catheter (10) is connected to the handle (20), wherein a driving mechanism is disposed in the handle (20), and the driving mechanism is connected to the inner tube assembly (11), to drive the inner tube assembly (11) to axially move relative to the outer tube assembly (12).

## Patentansprüche

1. Herzklappenzufuhrkatheter (10), umfassend eine äußere Schlauchanordnung (12) und eine innere Schlauchanordnung (11), wobei die äußere Schlauchanordnung (12) eine Stent-Segmenthülle (121) umfasst, die konfiguriert ist, um eine Klappe aufzunehmen, und einen äußeren Zufuhrsegmentschlauch (122), der mit einem proximalen Ende der Stent-Segmenthülle (121) verbunden ist, umfasst; die innere Schlauchanordnung (11) in einem Hohlraum der äußeren Schlauchanordnung (12) angeordnet ist und die innere Schlauchanordnung (11) in Bezug auf die äußere Schlauchanordnung (12) axial beweglich ist; und der äußere Zufuhrsegmentschlauch (122) einen Polymerschlauch (1221) und eine hohle röhrenförmige Struktur (1222) umfasst, die in dem Polymerschlauch (1221) angeordnet ist;
wobei die hohle röhrenförmige Struktur (1222) aus einem Metallrohr geschnitten ist und die hohle röhrenförmige Struktur (1222) eine Vielzahl von hohlen Abschnitten (12221), die in einer axial beabstandeten Weise angeordnet sind, und Verbindungsabschnitte (12222) umfasst, die jeweils zwischen zwei benachbarten der Vielzahl von hohlen Abschnitten (12221) gebildet sind; eine gerade Anzahl von Verbindungsrippen (12221a) und eine gerade Anzahl von hohlen Schlitzen auf jedem hohlen Abschnitt (12221) in einer Umfangsrichtung des hohlen Abschnitts (12221) angeordnet sind; jede der Verbindungsrippen (12221a) zwischen zwei der Vielzahl von hohlen Schlitzen angeordnet ist; und die Verbindungsrippen (12221a) konfiguriert sind, um zwei axial benachbarte der Verbindungsabschnitte (12222) zu verbinden;
wobei jeder der hohlen Schlitze W-förmig ist; jeder der hohlen Abschnitte (12221) ferner ein Klemmstück (12221b) und eine Haltenut (12221c) umfasst, die entlang der Umfangsrichtung angeordnet sind; die Haltenut (12221c) ein Teil eines entsprechenden der hohlen Schlitze ist; sich das Klemmstück (12221b) und die Haltenut (12221c) auf einer Mittelsenkrechten einer Linie befinden, die zwei benachbarte der Verbindungsrippen (12221a) verbindet; das Klemmstück (12221b) in der Haltenut (12221c) in der Umfangsrichtung des hohlen Abschnitts (12221) begrenzt ist; und wenn benachbarte der Verbindungsabschnitte (12222) eine relative Drehung ausführen, sich das Klemmstück (12221b) in Bezug auf die Begrenzungsnut (52) bewegt und hat eine Begrenzungsposition aufweist;
wobei Linien (W1, W2), die jeweils zwei gegenüberliegende Verbindungsrippen (12221a) an einem jeweiligen der hohlen Abschnitte (12221) an zwei Enden von jedem der Verbindungsabschnitte (12222) verbinden, Vorsprünge aufweisen, die sich in einem Querschnitt (C) des Verbindungsabschnitts (12222) schneiden;
wobei die Abstände zwischen den Vorsprüngen axial benachbarter Verbindungsrippen (12221a) auf einer gleichen vertikalen Projektionsebene gleich sind und die Verbindungslinien der Vielzahl von axial verteilten Verbindungsrippen (12221a) spiralförmig sind.

2. Herzklappenzufuhrkatheter (10) nach Anspruch 1, wobei die Stent-Segmenthülle (121) eine metallverstärkte Hülle (1211), einen elastischen Verlängerungsabschnitt (1212), der mit einem distalen Ende der metallverstärkten Hülle (1211) verbunden ist, und eine Polymerbeschichtung umfasst, die auf eine Außenseite der metallverstärkten Hülle (1211) und des elastischen Verlängerungsabschnitts (1212) aufgetragen ist; wobei die metallverstärkte Hülle (1211) eine hohle Struktur mit spiralförmigen Verstärkungsrippen aufweist.

3. Herzklappenzufuhrkatheter (10) nach Anspruch 1, wobei die gerade Zahl zwei ist.

4. Herzklappenzufuhrkatheter (10) nach Anspruch 1, wobei die Ränder von jedem der hohlen Schlitze alle bogenförmig gekrümmt sind.

5. Herzklappenzufuhrkatheter (10) nach Anspruch 1, wobei der äußere Schlauch des äußeren Zufuhrsegmentschlauchs (122) ferner eine metallgeflochtene röhrenförmige Struktur umfasst und sowohl die metallgeflochtene röhrenförmige Struktur als auch die hohle röhrenförmige Struktur (1222) in einer Schlauchwand des Polymerschlauchs (1221) angeordnet sind;
wobei die metallgeflochtene röhrenförmige Struktur in allen Abschnitten des äußeren Zufuhrsegmentschlauchs (122) angeordnet ist und die hohle röhrenförmige Struktur (1222) in einigen der Abschnitte des äußeren Zufuhrsegmentschlauchs (122) in der Nähe der Stent-Segmenthülle (121) angeordnet ist.

6. Herzklappenzufuhrkatheter (10) nach Anspruch 1, wobei der äußere Schlauch des äußeren Zufuhrsegmentschlauchs (122) ferner eine metallgeflochtene röhrenförmige Struktur umfasst und sowohl die metallgeflochtene röhrenförmige Struktur als auch die hohle röhrenförmige Struktur (1222) in einer Schlauchwand des Polymerschlauchs (1221) angeordnet sind;
wobei der Polymerschlauch (1221) ein erstes Segment und ein zweites Segment umfasst, die miteinander verbunden sind, die hohle röhrenförmige Struktur (1222) in dem ersten Segment angeordnet ist und das zweite Segment eine größere Härte als eine Härte des ersten Segments aufweist.

7. Herzklappenzufuhrkatheter, umfassend einen Griff (20) und den Zufuhrkatheter (10) nach einem der Ansprüche 1 bis 6, wobei der Zufuhrkatheter (10) mit dem Griff (20) verbunden ist, wobei ein Antriebsmechanismus in dem Griff (20) angeordnet ist und der Antriebsmechanismus mit der inneren Schlauchanordnung (11) verbunden ist, um die innere Schlauchanordnung (11) anzutreiben, um sich axial in Bezug auf die äußere Schlauchanordnung (12) zu bewegen.

## Revendications

1. Cathéter de pose de valvule cardiaque (10), comprenant un ensemble tube externe (12) et un ensemble tube interne (11), dans lequel l'ensemble tube externe (12) comprend une gaine pour segment d'endoprothèse (121) configurée pour accueillir une valvule et un tube externe pour segment de pose (122) relié à une extrémité proximale de la gaine pour segment d'endoprothèse (121) ; l'ensemble tube interne (11) est agencé dans une cavité de l'ensemble tube externe (12), et l'ensemble tube interne (11) est axialement mobile par rapport à l'ensemble tube externe (12) ; et le tube externe pour segment de pose (122) comprend un tube en polymère (1221) et une structure tubulaire creuse (1222) agencée dans le tube en polymère (1221) ;
dans lequel la structure tubulaire creuse (1222) est découpée dans un tube métallique, et la structure tubulaire creuse (1222) comprend une pluralité de parties creuses (12221) agencées de manière espacée axialement et des parties de liaison (12222) formées chacune entre deux parties creuses adjacentes de la pluralité de parties creuses (12221) ; un nombre pair de nervures de liaison (12221a) et un nombre pair de fentes creuses sont disposées sur chaque partie creuse (12221) dans une direction circonférentielle de la partie creuse (12221) ; chacune des nervures de liaison (12221a) est agencée entre deux fentes creuses de la pluralité de fentes creuses ; et les nervures de liaison (12221a) sont configurées pour relier deux parties de liaison axialement adjacentes des parties de liaison (12222) ;
dans lequel chacune des fentes creuses est en forme de W ; chacune des parties creuses (12221) comprend en outre une pièce de serrage (12221b) et une rainure de retenue (12221c) qui sont agencées le long de la direction circonférentielle ; la rainure de retenue (12221c) fait partie d'une fente creuse correspondante des fentes creuses ; la pièce de serrage (12221b) et la rainure de retenue (12221c) sont agencées sur une perpendiculaire médiane d'une ligne reliant deux nervures de liaison adjacentes des nervures de liaison (12221a) ; la pièce de serrage (12221b) est limitée dans la rainure de retenue (12221c) dans la direction circonférentielle de la partie creuse (12221) ; et lorsque des parties de liaison adjacentes des parties de liaison (12222) effectuent une rotation relative, la pièce de serrage (12221b) se déplace par rapport à la rainure de limitation (52) et a une position de limitation ;
dans lequel les lignes (W1, W2) reliant chacune deux nervures de liaison (12221a) opposées sur une partie creuse respective des parties creuses (12221) à deux extrémités de chacune des parties de liaison (12222) ont des projections qui se croisent sur une section transversale (C) de la partie de liaison (12222) ;
dans lequel les distances entre les projections de nervures de liaison (12221a) axialement adjacentes sur un même plan de projection vertical sont égales, et les lignes reliant la pluralité de nervures de liaison (12221a) qui sont réparties axialement sont en spirale.

2. Cathéter de pose de valvule cardiaque (10) selon la revendication 1, dans lequel la gaine pour segment d'endoprothèse (121) comprend une gaine renforcée par du métal (1211), une section d'extension élastique (1212) reliée à une extrémité distale de la gaine renforcée par du métal (1211), et un revêtement polymère enduit sur un côté extérieur de la gaine renforcée par du métal (1211) et de la section d'extension élastique (1212) ; dans lequel la gaine renforcée par du métal (1211) a une structure creuse avec des nervures de renfort en spirale.

3. Cathéter de pose de valvule cardiaque (10) selon la revendication 1, dans lequel le nombre pair est deux.

4. Cathéter de pose de valvule cardiaque (10) selon la revendication 1, dans lequel les bords de chacune des fentes creuses sont tous des courbes en forme d'arc.

5. Cathéter de pose de valvule cardiaque (10) selon la revendication 1, dans lequel le tube externe pour segment de pose (122) comprend en outre une structure tubulaire tressée en métal, et la structure tubulaire tressée en métal et la structure tubulaire creuse (1222) sont toutes deux agencées dans une paroi tubulaire du tube en polymère (1221) ;
dans lequel la structure tubulaire tressée en métal est agencée dans toutes les sections du tube externe pour segment de pose (122), et la structure tubulaire creuse (1222) est agencée dans certaines des sections du tube externe pour segment de pose (122) près de la gaine pour segment d'endoprothèse (121).

6. Cathéter de pose de valvule cardiaque (10) selon la revendication 1, dans lequel le tube externe pour segment de pose (122) comprend en outre une structure tubulaire tressée en métal, et la structure tubulaire tressée en métal et la structure tubulaire creuse (1222) sont toutes deux agencées dans une paroi tubulaire du tube en polymère (1221) ;
dans lequel le tube en polymère (1221) comprend un premier segment et un second segment qui sont reliés l'un à l'autre, la structure tubulaire creuse (1222) est agencée dans le premier segment, et le second segment a une dureté supérieure à une dureté du premier segment.

7. Système de pose de valvule cardiaque, comprenant une poignée (20) et le cathéter de pose (10) selon l'une quelconque des revendications 1 à 6, le cathéter de pose (10) est relié à la poignée (20), dans lequel un mécanisme d'entraînement est agencé dans la poignée (20), et le mécanisme d'entraînement est relié à l'ensemble tube interne (11), pour entraîner l'ensemble tube interne (11) à se déplacer axialement par rapport à l'ensemble tube externe (12).
